Europäisches Patentamt

European Patent Office

Office européen des brevets

(19)

(11) Veröffentlichungsnummer **0 088 832**
**B1**

(12)

# EUROPÄISCHE PATENTSCHRIFT

(45) Veröffentlichungstag der Patentschrift:
**11.09.85**

(51) Int. Cl.⁴: **A 61 F 13/00**

(21) Anmeldenummer: **82200329.9**

(22) Anmeldetag: **13.03.82**

(54) **Schnelldruckverband.**

(43) Veröffentlichungstag der Anmeldung:
**21.09.83 Patentblatt 83/38**

(45) Bekanntmachung des Hinweises auf die Patenterteilung:
**11.09.85 Patentblatt 85/37**

(84) Benannte Vertragsstaaten:
**AT BE CH DE FR GB IT LI LU NL SE**

(56) Entgegenhaltungen:
**DE - C - 316 451**
**DE - C - 660 433**
**DE - U - 7 340 366**
**FR - A - 867 247**
**FR - A - 1 099 632**
**US - A - 2 152 922**
**US - A - 2 936 759**
**US - A - 3 774 614**

(73) Patentinhaber: **Vujko, Milenko, Rychenbergstrasse 22, CH-8400 Winterthur (CH)**

(72) Erfinder: **Vujko, Milenko, Rychenbergstrasse 22, CH-8400 Winterthur (CH)**

(74) Vertreter: **Feldmann, Clarence Paul et al, c/o Patentanwaltsbüro FELDMANN AG Postfach Kanalstrasse 17, CH-8152 Glattbrugg (CH)**

## Beschreibung

Die vorliegende Erfindung betrifft einen Schnelldruckverband bestehend aus einer eine zu dichtenden Wunde deckenden und schützenden Folie mit einem auf der Folie befindlichen blutdichtenden Ringwulst, sowie aus mit der Folie verbundenen Befestigungsmitteln.

Das Anlegen eines Druckverbandes ist eine lebensrettende Maßnahme, die schnell aber auch korrekt erfolgen muß. Dabei soll einerseits durch Druck das verletzte Blutgefäß zugepreßt werden, damit die Blutung gestillt wird, andererseits dürfen aber nicht unnötig weitere Gefäße abgepreßt oder gar Nerven eingeklemmt werden. Die bis heute in den meisten Notverbandskasten vorhandenen Verbandsmaterialien verlangen vom Anwender ein, meist mangels Praxis, nicht vorhandenes Können.

Entsprechend groß ist die Zahl der durch unsachgemäßes Anlegen eines Druckverbandes verursachten Schäden.

Aus der DE-C-660 433 ist ein Schnelldruckverband gemäß dem Oberbegriff des Anspruches 1 bekannt, der insbesondere für größere Verletzungen gedacht ist und eine dichtende Lage aus einem und einen Ringwulst aus einem anderen weichen, blutdichtenden Material aufweist. Der Ringwulst ist mit einer Klebeschicht zur Dichtung versehen. Die dichtende Lage läuft in Gurtenden aus. Der Bereich innerhalb des Ringwulstes ist mit einem blutstillenden Kissen versehen. Dieser Schnelldruckverband ist als solcher ungeeignet, weil die blutende Wunde selbst lediglich durch die Verklebung zu einem blutdichtenden Druckverband werden würde, was jedoch voraussetzt, daß überhaupt eine Verklebung möglich ist. Dies ist jedoch auf einer blutverschmierten Haut in der Nähe einer stark blutenden Wunde kaum wahrscheinlich. Folglich müßte man den Verschlußgurt derart fest zuziehen, daß wiederum eine gefährliche, vollständige Abschnürung erfolgt.

Die DE-U-7 340 366 beschreibt eine Schutzvorrichtung zum berührungsfreien Abdecken einer Körperstelle. Hierbei handelt es sich um ein einstückiges flächiges Gebilde aus einem plastisch verformbaren Kunststoff, welcher relativ fest ist. Der mittlere Bereich ist nach oben gewölbt und soll ein Verkleben mit der Wunde verhindern.

Die vorliegende Erfindung stellt sich die Aufgabe, den Schnelldruckverband der eingangs genannten Art so auszubilden, daß er auch vom Laien gefahrlos angelegt werden kann.

Diese Aufgabe wird dadurch gelöst, daß die Folie und der als Druckring ausgebildete Ringwulst einstückig aus ein und demselben gummielastischen Kunststoff gefertigt sind, wobei der Ringwulst eine spitze Querschnittsform aufweist, und die Befestigungsmittel aus mit der Folie verbundenen Gazen bestehen.

Dank dieser Ausbildung ist eine optimale Anpassung des Ringwulstes an die Körperpartie in jedem Wundbereich gegeben und die spitze Querschnittsform des Ringwulstes führt zu einem relativ hohen spezifischen Druck der jedoch durch Elastizität des gummielastischen Materials nicht zu einer weiteren Verletzung des Verunfallten führen kann. Die an der Folie befestigten Gazen ermöglichen ein schnelles Anlegen des Schnelldruckverbandes.

Vorteilhaft ist es, wenn die Folie ein Streifen ist, so daß beidseitig des Ringwulstes je eine Folienlasche verbleibt, an denen die Gazen zum Anlegen des Schnelldruckverbandes angebracht sind.

Auf diese Weise kann der Schnelldruckverband einfach aufgebracht werden, ohne daß die mit der Wunde in Verbindung kommenden keimfreien Teile mit der Hand berührt werden müssen, und weil so der Zug der Gazen einen gleichmäßigen verteilten Druck auf den blutdichtenden Ringwulst aufbringt. Für größere Schnelldruckverbände ist es sinnvoll, wenn innerhalb des blutdichtenden Ringwulstes ein zweiter stützender Ring einstückig an der Folie angeformt ist.

Dadurch wird vermieden, daß die Folie direkt auf die Wunde zu liegen kommt und dadurch der spezifische Druck des blutdichtenden Ringwulstes durch den Flächendruck der Folie verringert wird.

In der Zeichnung sind einige Ausführungsbeispiele des Erfindungsgegenstandes dargestellt. Es zeigt

Fig. 1 einen erfindungsgemäßen Schnelldruckverband in der Aufsicht und

Fig. 2 im Schnitt;

Fig. 3 einen Schnelldruckverband mit einem stützenden Ringwulst innerhalb des dichtenden Ringwulstes, in der Aufsicht und

Fig. 4 im Schnitt;

Fig. 5 einen Schnelldruckverband entsprechend Fig. 3, wobei jedoch der stützende Ringwulst zu einem Ring von Stützpunkten reduziert ist, in der Aufsicht und

Fig. 6 wiederum im Schnitt;

Fig. 7 zeigt eine Variante mit kreuzförmig angebrachten Laschen und

Fig. 8 ein Beispiel mit genau zylindrischem dichtendem Ringwulst.

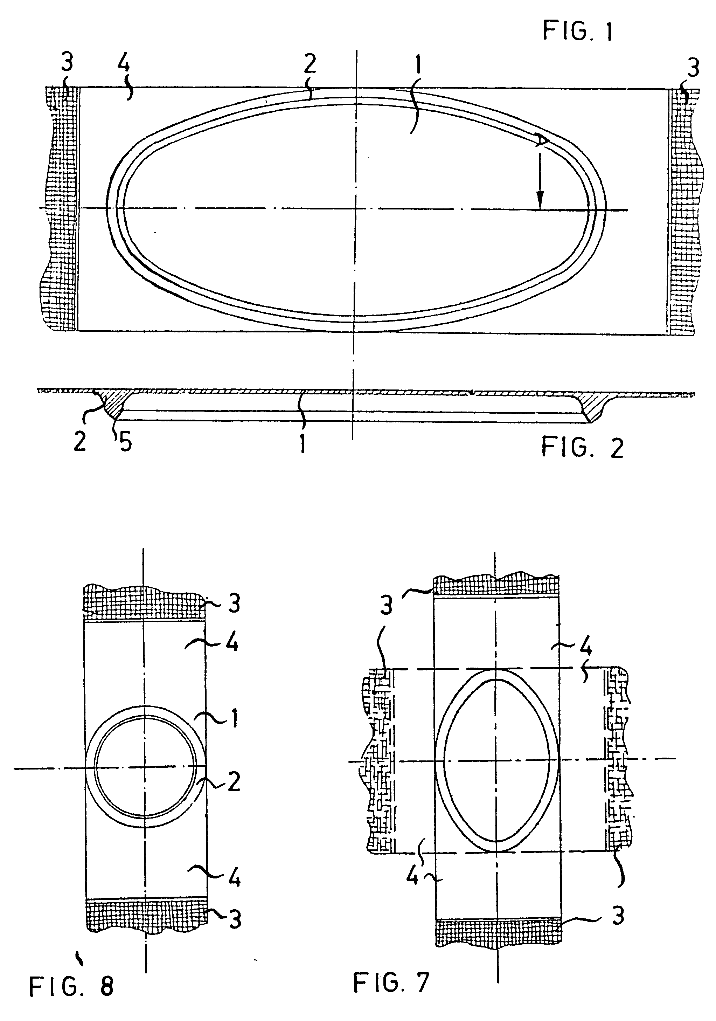

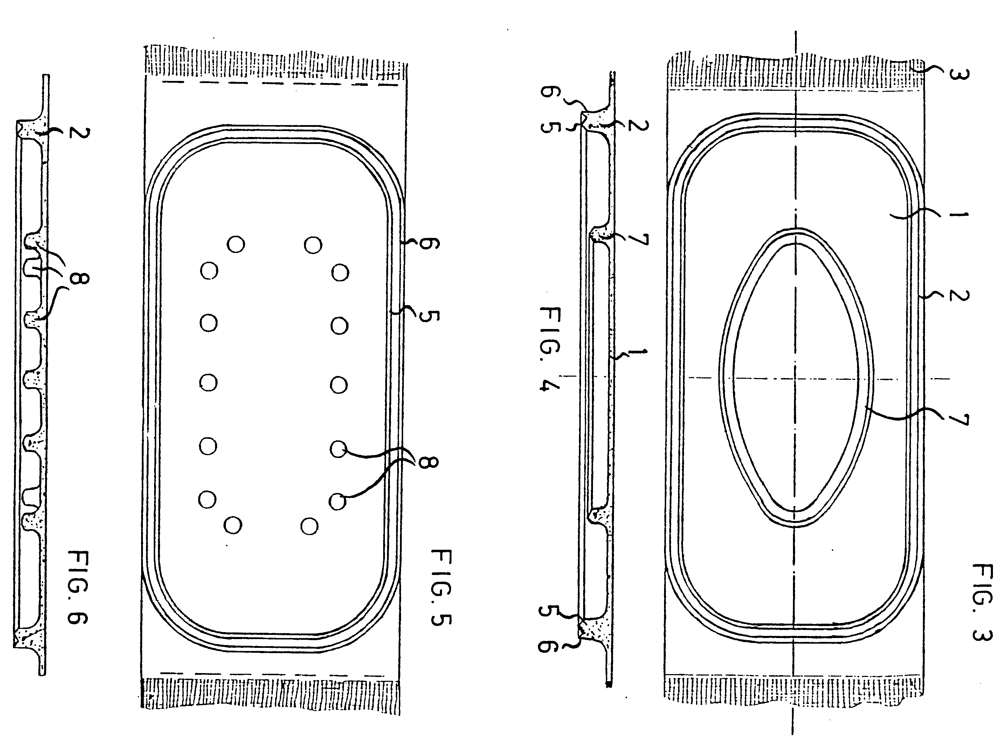

In Fig. 1 ist ein Schnelldruckverband dargestellt aus einer streifenförmigen gummielastischen Folie 1. Die Folie 1 und ein darauf befindlicher Ringwulst 2 sind einstückig ausgebildet. Beidseitig des länglichen Ringwulstes 2 stehen Laschen 4 in Verlaufrichtung des Folienstreifens 1 vor. An diesen Laschen sind Gazen 3 angebracht, mit denen der Schnelldruckverband angelegt werden kann.

Der Querschnitt des Ringwulstes ist in Fig. 2 deutlich ersichtlich. Er weist eine relativ breite Basis auf, die gerundet in eine kurze Partie etwa gleichbleibender Dicke übergeht, bevor der Querschnitt in eine scharfkantige Spitze 5 ausläuft. Diese Form bewirkt die nötige Festigkeit, damit der Druck des Schnelldruckverbandes auf die Spitze übertragen wird und ein relativ hoher spezifischer Druck erzeugt werden kann, ohne daß die Spitze angeknickt wird und dadurch ein Flächendruck mit erheblich geringerem spezifischen Druck auftritt.

Die Fig. 3 und 4 zeigen einen besonders großen Schnelldruckverband. Der blutdichtende Ringwulst hat hier eine Querschnittsform, die in zwei Spitzen 5 und 6 ausläuft. Ein derart großer Schnelldruckverband wird nicht nur durch die Gazen 3 an den Laschen 4 gehalten, sondern meist durch eine zusätzliche elastische Binde oder anderem Verbandsmaterial, welches gerade zur Verfügung steht. Bei einem derart großen Schnelldruckverband ist die Wahrscheinlichkeit, daß dieser über Wölbungen oder Rundungen hinweg verläuft groß. Dabei würde die Folie 1 direkt auf die Wunde zu liegen kommen, wenn nicht ein stützender Ringwulst 7 dies verhindern würde. Dabei kommt es nicht darauf an, die Folie von der Wunde fern zu halten. Hierzu besteht keine Veranlassung, denn erstens ist der Schnelldruckverband lediglich ein kurzzeitig zu verwendender Notverband und zweitens ist das Material vollständig steril. Wichtig ist lediglich, daß durch den Flächendruck auf die Wunde der erforderliche Druck auf den blutdichtenden Ringwulst sich vermindern kann. Dies soll der stützende Ringwulst 7 vermeiden, wobei er jedoch gegebenenfalls selber als blutdichtender Ringwulst wirken kann. Die Höhe des stützenden Ringwulstes ist geringer als jene des äußeren blutdichtenden Ringwulstes, so daß der stützende Ringwulst lediglich unter bestimmten Umständen eine Funktion übernimmt.

Die stützende Funktion kann der innere Ringwulst auch ausüben, wenn er, wie in den Fig. 5 und 6 dargestellt, teilweise unterbrochen oder gar auf ringförmig angeordnete Stützpunkte 8 reduziert ist.

Für gewisse Körperpartien kann es sinnvoll sein, wenn die Folie 1 zwei kreuzweise angeordnete Bänder bildet, so daß vier Laschen 4 zur Verfügung stehen an denen Gazen 3 zum Anlegen des Schnelldruckverbandes anschließen. Die Verbindung zwischen der Folie 1 und den Gazen 3 kann mittels Kleben erfolgen oder aber bei der Herstellung im Kunststoff eingelassen werden (Fig. 7).

Abschließend sei lediglich noch darauf hingewiesen, daß der Ringwulst 2 eine beliebige geschlossene Form sein kann, die wie in den meisten Beispielen oval oder wie in Fig. 8 dargestellt rund sein kann.

## Patentansprüche

1. Schnelldruckverband bestehend aus einer eine zu dichtenden Wunde deckenden und schützenden Folie (1) mit einem auf der Folie befindlichen blutdichtenden Ringwulst (2), sowie aus mit der Folie verbundenen Befestigungsmitteln (3), dadurch gekennzeichnet, daß die Folie (1) und der als Druckring ausgebildete Ringwulst (2) einstückig aus ein und demselben gummielastischen Kunststoff gefertigt sind, wobei der Ringwulst (2) eine spitze (5) Querschnittsform aufweist, und die Befestigungsmittel aus mit der Folie (1) verbundenen Gazen (3) bestehen.

2. Schnelldruckverband nach Anspruch 1, dadurch gekennzeichnet, daß die Folie (1) ein Streifen ist, an welchem beidseitig des Ringwulstes (2) je eine Folielasche (4) verbleibt, an denen die Gazen (3) angebracht sind.

3. Schnelldruckverband nach Anspruch 1, dadurch gekennzeichnet, daß innerhalb des Ringwulstes (2) ein zweiter stützender Ring (7) einstückig an der Folie (1) angeformt ist.

4. Schnelldruckverband nach Anspruch 4, dadurch gekennzeichnet, daß der stützende Ring (7) in die Auflage verkleinernde Unterbrüche aufgeteilt ist.

## Claims

1. High speed compression bandage consisting of a film (1) for covering and protecting a wound with a bloodsealing torus (2) located on the film, as well as fastening means (3) connected to the film, characterized in that the film (1) and the torus (2) constructed as a pressure ring are made in one piece from the same rubber-elastic plastic, the torus (2) having an acute (5) cross-sectional shape and the fastening means comprise gauze (3) connected to the film (1).

2. High speed compression bandage according to claim 1, characterized in that the film (1) is a strip, on which a film flap (4) is left on either side of the torus (2) and on same are provided the gauzes (3).

3. High speed compression bandage according to claim 1, characterized in that a second supporting ring (7) shaped in one piece on film (1) is provided within the torus (2).

4. High speed compression bandage according to claim 1, characterized in that the supporting ring (7) has interruptions so as to reduce the supporting action.

## Revendications

1. Bandage compressif rapide formé d'une feuille (1) couvrant et protégeant une plaie à rendre étanche et d'un bourrelet annulaire (2) situé sur la feuille, assurant l'étanchéité au sang, ainsi que de moyens de fixation (3) reliés à la feuille, caractérisé en ce que la feuille (1) et le bourrelet annulaire (2) conçu sous forme d'anneau de compression sont fabriqués d'une seule pièce en une même matière synthétique à la façon du caoutchouc, le bourrelet annulaire (2) présentant une forme de section pointue (5) et les moyens de fixation étant formés de gazes (3) reliées à la feuille (1).

2. Bandage compressif rapide selon la revendication 1, caractérisé en ce que la feuille (1) est une bande sur laquelle il reste, de chacun des deux côtés du bourrelet annulaire (2), une patte de feuille (4), les gazes (3) étant disposées sur ces pattes.

3. Bandage compressif rapide selon la revendication 1, caractérisé en ce qu'à l'intérieur du bourrelet annulaire (2), un deuxième anneau de soutien (7) est formé solidairement sur la feuille (1).

4. Bandage compressif rapide selon la revendication 4, caractérisé en ce que l'anneau de soutien (7) est divisé par des interruptions rendant plus petite la surface d'application.

0 088 832

FIG. 1

FIG. 2

FIG. 8

FIG. 7

FIG. 3

FIG. 4

FIG. 5

FIG. 6